# Europäisches Patentamt

# European Patent Office

# Office européen des brevets

(11) Numéro de publication: **0 146 416**
**B1**

(19)

(12) **FASCICULE DE BREVET EUROPÉEN**

(45) Date de publication du fascicule du brevet:
**02.08.89**

(21) Numéro de dépôt: **84401768.1**

(22) Date de dépôt: **06.09.84**

(51) Int. Cl.⁴: **C 12 N 15/00,** C 12 N 1/20 //
(C12N1/20, C12R1:19)

(54) **Vecteur modifié par une partie au moins, sinon la totalité, du gène lam B et micro-organismes transformés par ce vecteur, et rendus aptes à synthétiser une protéine de membrane externe déterminée, codée par un insérat également contenu dans ledit vecteur.**

(30) Priorité: **06.09.83 FR 8314236**

(43) Date de publication de la demande:
**26.06.85 Bulletin 85/26**

(45) Mention de la délivrance du brevet:
**02.08.89 Bulletin 89/31**

(84) Etats contractants désignés:
**AT BE CH DE FR GB IT LI LU NL SE**

(56) Documents cité:
**EP-A-0 012 078**
**US-A-4 336 336**

(73) Titulaire: **INSTITUT PASTEUR, 25/28, rue du
Docteur Roux, F-75015 Paris (FR)**
Titulaire: **INSTITUT NATIONAL DE LA SANTE ET
DE LA RECHERCHE MEDICALE (INSERM), 101,
rue de Tolbiac, F-75654 Paris Cédex 13 (FR)**
Titulaire: **CENTRE NATIONAL DE LA RECHERCHE
SCIENTIFIQUE (CNRS), 15, Quai Anatole France,
F-75007 Paris (FR)**

(72) Inventeur: **Hofnung, Maurice, 19 bis, rue Bargue,
F-75015 Paris (FR)**
Inventeur: **Bouges- Bocquet, Bernadette, 2, rue du
Harpon, F-92290 Chatenay (FR)**
Inventeur: **Guesdon, Jean- Luc, 12, rue du Hameau,
F-75015 Paris (FR)**

(74) Mandataire: **Gutmann, Ernest, S.C. Ernest
Gutmann - Yves Plasseraud 67, boulevard
Haussmann, F-75008 Paris (FR)**

LIBER, STOCKHOLM 1989

## Description

L'invention est relative à des vecteurs perfectionnés modifiés par au moins une partie du gène lam B, sinon la totalité de celui-ci, ce vecteur pouvant à son tour être modifié par un insérat constitué par un fragment déterminé de nucleotides codant pour une séquence déterminée d'aminoacides.

L'invention est encore relative à des souches de micro-organismes, plus particulièrement de E. coli, transformées par un tel vecteur et rendues aptes à synthétiser une protéine hybride contenant ladite séquence déterminée d'aminoacides et à la transporter à la surface externe de leurs membranes.

Des vecteurs appartenant à ce domaine technique ont déjà été décrits dans des publications et brevets antérieurs. On rappellera en particulier que l'on avait déjà décrit dans le brevet francais n° 7 833 498/2 442 271 déposé le 27 Novembre 1978 un vecteur porteur du promoteur de l'opéron lactose et d'au moins un fragment du gène Z de ce même opéron, caractérisé par le fait qu'au moins une séquence, plus particulièrement une séquence comportant la partie proximale du gène lam B se trouve insérée dans ce vecteur, plus particulièrement dans le susdit fragment de gène Z ou immédiatement en aval de celui-ci. La longueur de cette séquence du gène lam B devait être suffisante pour permettre la production par le micro-organisme transformé, notamment E. coli, sous le contrôle du promoteur de l'opéron lactose d'une protéine hybride transportée dans la membrane externe de ces cellules et contenant des séquences d'acides aminés contenues dans le récepteur du phage λ et la β-galactosidase et respectivement codées par lam B et le gène S. Dans certains cas, la protéine hybride peut être excrétée, dans le milieu de culture dudit micro-organisme transformé.

Il a cependant été constaté que la production de la protéine hybride de membrane externe n'allait pas sans difficultés, que la réalisation de la culture des micro-organismes transformés dans des conditions permettant d'assurer la transcription et la traduction de la séquence correspondante de nucléotides, pouvait s'accompagner soit d'un certain niveau d'instabilité, soit d'une certaine toxicité de la protéine hybride synthétisée, pouvant entraîner la lyse d'une proportion importante des cultures cellulaires, soit des deux à la fois.

La présente invention a pour but de remédier au moins en partie à ces difficultés, notamment de fournir des vecteurs du type sus-indiqué, contenant également un insérat formé d'une séquence nucléotidique codant pour une séquence d'acides aminés déterminée, dont on désire induire la synthèse dans un micro-organisme approprié, plus particulièrement E. coli, dans des conditions telles que la protéine exprimée correspondante présente une stabilité plus satisfaisante et que les colonies bactériennes transformées puissent se développer et fournir des générations successives de micro-organismes transformés et producteurs de ladite protéine hybride à la surface externe de leurs cellules, tout en contrôlant l'éventuelle lyse d'une partie des cellules, dans des proportions compatibles avec la production de colonies suffisamment importantes de cellules transformées.

L'invention résulte de la découverte que des cellules modifiées, comportant sur leurs membranes externes, des protéines hybrides contenant elles-mêmes une séquence d'acides aminés déterminée, répondant aux conditions sus-indiquées peuvent être obtenues par transformation préalable de ces cellules avec un vecteur modifié, dans lequel le fragment nucléotidique codant pour la séquence déterminée d'aminoacides est lui-même inséré dans le gène lam B placé sous le contrôle d'un promoteur suffisamment fort pour permettre la transcription et la traduction de la totalité de la séquence définie par les éléments correspondants du gène lam B (ou de la partie de gène lam B) et du fragment déterminé de nucléotides, lorsque les conditions suivantes sont respectées.

Le vecteur modifié selon l'invention est modifié par une séquence nucléotidique contenant un insérat déterminé de nucléotides codant pour une séquence déterminée d'aminoacides, cette séquence nucléotidique étant placée sous le contrôle d'un promoteur suffisamment fort pour permettre la transcription et la traduction essentiellement de la totalité de ladite séquence nucléotidique dans une souche de E. coli préalablement transformée par ledit vecteur modifié. Le vecteur modifié selon l'invention est caractérisé en ce que la partie proximale de ladite sequence nucléotidique, vis-à-vis dudit promoteur et en amont dudit insérat déterminé de nucléotides, contient la partie antérieure du gène lam B codant pour au moins les 180 premiers aminoacides de la partie N-terminale de de la protéine correspondante, et en aval dudit insérat déterminé, la partie postérieure du gène lam B, codant pour au moins les 20 derniers aminoacides de la partie C-terminale de la protéine correspondante, lesdites parties antérieure et postérieure du gène lam B ainsi que le susdit fragment de nucléotides étant respectivement en phase avec le promoteur, afin que la protéine hybride résultant de la traduction de ladite séquence sous le contrôle dudit promoteur contienne, d'une part, respectivement au moins les 180 premiers et au moins les 20 derniers aminoacides de la protéine codée par le gène lam B et, d'autre part, la susdite séquence déterminée d'aminoacides.

Il s'est en effet avéré que la présence, en aval du fragment déterminé du nucléotide codant pour la séquence d'acides aminés recherchés, de la partie postérieure du gène lam B codant pour au moins les 20 derniers aminoacides de la partie C-terminale de la protéine correspondante (ou comme on le dira dans ce qui suit, pour la simplicité du langage, de la partie C-terminale du gène lam B), était une condition essentielle à l'obtention de bonnes conditions de transformation. L'absence de l'extrémité C-terminale du gène lam B, voire son altération, manifeste en effet par un considérable accroissement de la toxicité de la protéine codée par le vecteur ainsi modifié à l'égard du micro-organisme.

La réalisation d'un vecteur contenant cette partie C-terminale du gène lam B, dans les conditions qui ont été énoncées, va à l'encontre de nombreuses tentatives décrites dans la littérature, pour produire des vecteurs mettant en jeu des fusions de séquences constituées à partir de fragments de gène lam B et de l'insérat codant pour la séquence d'acides aminés dont la transcription et la traduction sont recherchées, dans lesquelles les fragments de gène lam B sont en général toujours placés en amont seulement de l'insérat et sont le plus souvent une partie N-terminale du gène lam B présumées contenir la séquence signal et les informations nécessaires au transport de la protéine hybride à travers la membrane externe des cellules.

La condition relative à la longueur minimum de la partie inférieure du gène lam B, en aval de l'insérat, est importante également. On constate en effet que la condition exprimée plus haut, que cette partie antérieure, codant pour au moins les 180 premiers aminoacides de la partie N-terminale de la protéine correspondante (ou encore la partie N-terminale du gène lam B), est importante aussi du point de vue de la stabilité de la protéine synthétisée. La protéine hybride dont la synthèse est induite par des vecteurs, dans lesquelles la partie N-terminale du gène B serait plus courte en amont de l'insérat conduirait à des protéines hautement instables, comme cela peut être détecté par des essais de caractérisation des protéines induites en présence d'un marqueur radioactif.

De préférences, les susdites parties antérieure et postérieure du gène lam B contiennent ensemble un nombre total suffisant de nucléotides pour que de telles parties, lorsqu'elles sont placées en phase sous le contrôle d'un promoteur tac à l'intérieur d'un plasmide le contenant, soient aptes à permettre l'obtention dans une souche de E. coli préalablement transformée par ce plasmide, d'une protéine stable et non léthale pour E. coli sur la membrane externe du micro-organisme.

Dans le cas de vecteurs mettant en jeu un promoteur tac (par exemple tel que décrit dans l'article de H.A. DE BOER et Coll. paru dans Proc. Natl. Acad. Sci. USA, Vol. 80 pp. 21 - 25, janvier 1983) la toxicité ou la léthalité de la protéine hybride synthétisée par le micro-organisme transformé peut être appréciée dans les conditions suivantes. Avantageusement, le microorganisme utilisé consiste en un mutant de E. coli, initialement résistant au phage et/ou initialement incapable de croître spontanément dans un milieu contenant une maltodextrine. On sait en effet que le gène lam B code pour le récepteur du phage λ. Le récepteur du phage se comporte également comme le feraient des pores dans la membrane cellulaire à l'égard des maltodextrines. La transformation de tels micro-organismes avec le vecteur selon l'invention (ou un vecteur contenant le gène lam B entier (sauvage)) restitue à ces mutants la sensibilité au phage λ et/ou la capacité de croître sur des maltodextrines notamment en présence d'un inducteur constitué par l'isopropylthiogalactoside (IPTG).

Dans les essais qui ont été réalisés dans le cadre de la présente invention, le mutant de E. coli utilisé était constitué de la souche $BB_1$ = thr leu tonB thi lacY1 recA dex5 metA supE, F' $lacI^{Q1}$ Z + Y + proAB +. Cette souche est dérivée de la souche pop 6510 et contient un épisome F' comportant lui-même une mutation $lacI^{Q1}$. Cette souche, transformee en outre par le plasmide pBBO, a été déposée à la C.N.C.M. de l'INSTITUT PASTEUR de Paris le 6 septembre 1983 sous le n° I-239. Des vecteurs préférés sont dérivés du plasmide pBBO obtenu par l'insertion d'un promoteur tac12 de DE BOER et Coll., inséré entre les sites EcoRI et BamHI du plasmide pHSF1 décrit par J.M. CLEMENT, D. PERRIN et J. HEDGPETH. 1982. Mol. Gen. G. 185, 302 - 310 juste avant la séquence de SHINE et DALGARNO du gène lam B.

La transformation de la susdite souche mutante par pBBO et la culture du micro-organisme transformé en présence d'IPTG $10^{-3}$M sur le milieu de culture solide de Luria, conduit à une surproduction de la protéine codée par lam B. Les bactéries transformées forment des colonies translucides qui se développent régulièrement jusqu'à confluence en l'absence sensible de lyse cellulaire.

La léthalité de certains vecteurs, par exemple ceux dans lesquels les 10 derniers triplets de l'extrémité C-terminale de lam B ont été modifiés, se manifeste par une sensibilité excessive à l'IPTG, pouvant entraîner la lyse de toutes les cellules après une seule génération.

A l'inverse, le défaut de stabilité des protéines synthétisées par un vecteur non conforme à l'invention peut être apprécié après immunopré-cipitation de la totalite des extraits cellulaires des cellules transformées recueillies après lyse ou rupture des parois cellulaires et marquées avec un acide aminé radioactif, par exemple la $^{35}$S-méthionine, selon la méthode décrite par ITO et Coll. (K. ITO, P. J. BASSFORD et J. R. BECKWITH, 1981, Cell 24, 707 - 717). La stabilité des protéines formées, notamment lorsque la traduction est interrompue de façon prématurée, se manifeste par une dégradation des protéines formées, de sorte que celles-ci ne peuvent rapidement plus être détectées par autoradiographie, dans des essais de fractionnement sur gel d'acrylamide - modifié par du dodécylsulfate de sodium (SDS) à 10 %. Cette instabilité s'observe notamment lorsque la partie proximale du gène lam B dans le vecteur selon l'invention est trop courte ou encore lorsque l'insérat, même introduit dans un site adéquat de lam B, entraîne un décalage de la phase de lecture de la partie C-terminale de la partie postérieure du gène lam B.

On appréciera donc que les tests qui viennent d'être décrits sont à l'origine de la détermination des conditions auxquelles doivent répondre les vecteurs selon l'invention, pour permettre la synthèse, dans des conditions optimales, de la protéine hybride de membrane externe. L'insertion d'un fragment déterminé de nucléotides codant pour une séquence déterminée d'aminoacides dans le gène lam B, dans les conditions qui ont été définies plus haut, conduit à des vecteurs aptes à rendre les cellules qu'ils transforment capables de se développer en présence d'IPTG, en formant des colonies qui ne lysent pas. En outre, les protéines immunoprécipitables persistent dans le temps, donc sont stables.

Les constatations qui ont été faites plus haut en ce qui concerne la capacité de la souche mutante transformée par pBBO de synthétiser des protéines hybrides se retrouvant dans les membranes externes des cellules, lorsqu'elles sont cultivées dans un milieu approprié en présence d'IPTG, se manifeste encore dans des cultures de cellules semblables modifiées par un vecteur dérivé de pBBO, par insertion dans celui-ci d'un insérat déterminé dans les conditions qui ont été définies en ce qui concerne l'invention. Là encore, on obtient des colonies translucides ou, le cas échéant, des colonies de moindre importance, cependant non lysées, et portant à l'extérieur de leurs membranes des protéines hybrides contenant le produit d'expression de l'insérat. Le fait même que ces colonies se soient développées dans les conditions sus-indiquées atteste d'ailleurs que le clonage et l'expression de l'insérat ont été réalisés. On peut également transformer des E. coli ne présentant pas les mutations sus-indiquées, étant alors entendu que la détection de la protéine hybride devrait alors mettre en jeu d'autres modes de détection, par exemple des anticorps préalablement formés contre la séquence d'acides aminés pour laquelle code l'insérat, ou contre une protéine contenant cette séquence d'acides aminés.

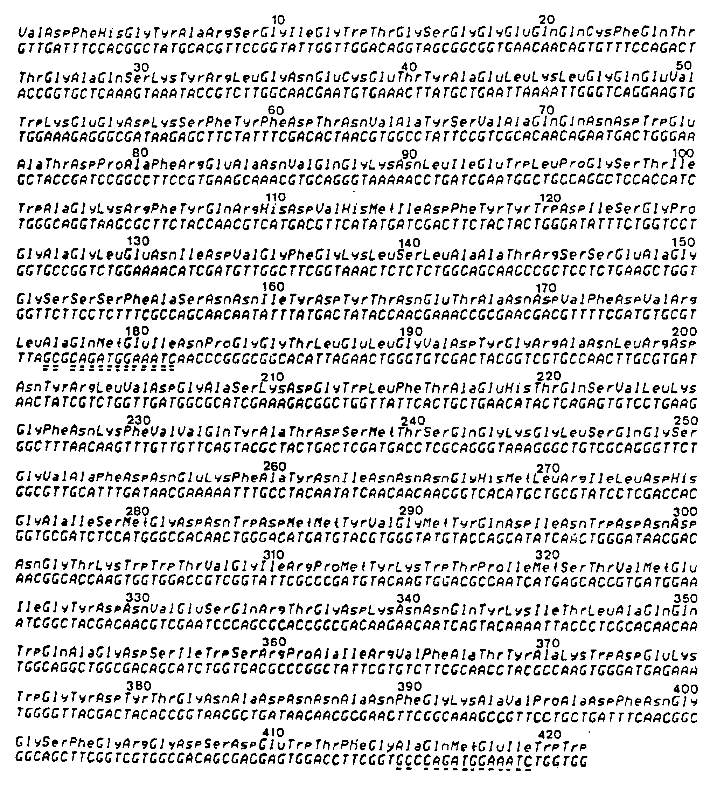

L'expérience montre que les meilleurs résultats sont obtenus lorsque les susdites parties antérieure et postérieure du gène lam B totalisent ensemble au moins 350 triplets de nucléotides sur les 420 triplets de ce gène, dont la séquence a été entièrement réalisée (fig. 1). Il est encore davantage préféré que les susdites parties antérieure et postérieure du gène lam B contiennent ensemble substantiellement la totalité des nucléotides du gène lam B. Les délétions réduites qui peuvent être envisagées, voire les modifications de certaines séquences des deux parties du gène lam B, doivent être telles qu'elles ne modifient pas sensiblement les propriétés globales de ce vecteur. La description des tests qui précèdent fournit à l'homme du métier les éléments qui lui permettront, en toutes circonstances, de tester celles des délétions ou modifications qui peuvent être réalisées, sans modifier l'ensemble des propriétés du vecteur selon l'invention. Il en sera encore de même en ce qui concerne la vérification de l'impact sur la bactérie ultérieurement transformée du choix du site d'insertion de l'insérat déterminé dans le vecteur et des longueurs relatives des parties antérieure et postérieure du gène lam B. D'une façon générale, notamment lorsque les parties antérieure et postérieure du gène lam B comprennent la quasi-totalité des nucléotides du gène intact, cette insertion pourra être effectuée entre le site 180 et le site 350 (la numérotation s'effectuant à partir du triplet codant pour le premier acide aminé du récepteur du phage λ).

En ce qui concerne le promoteur, on peut avoir recours à tout autre promoteur assez fort pour permettre la transcription et la traduction dans E. coli de la séquence totale, telle qu'elle a été définie plus haut. Bien que les promoteurs tac soient préférés les promoteurs lac et trp peuvent également être utilisés utilement.

Conformément aux constructions préférées de l'invention, les premiers triplets de nucléotides traduits sous le contrôle du promoteur choisi, sont constitués par les premiers triplets de la partie antérieure du gène lam B et, de préférence encore, le codon d'arrêt de la traduction se trouve placé immédiatement après le dernier acide aminé du récepteur du phage λ (trp en position 421).

Le vecteur peut être constitué par tout acide nucléique apte à transformer un E. coli. De préférence, il s'agit d'un plasmide tel que pBR322, le cas échéant d'un phage.

L'insertion de l'insérat au sein de la séquence du vecteur contenant le gène lam B, soit entier, soit délété des parties de moindre importance (par exemple la séquence 350 - 400) peut être réalisée par toute technique en soi connue. Dans le cas où le vecteur consiste dans un plasmide, on peut avoir recours à la linéarisation du du plasmide en utilisant des enzymes de restriction, la fixation de courtes séquences de nucléotides ("linkers") de préférence après bicaténarisation des extrémités de restriction à l'aide des nucléotides appropriés et en présence de la polymérase de Klenow, les "linkers" contenant le site de restriction correspondant aux extrémités cohésives de l'insérat que l'on veut introduire dans le site correspondant de lam B, le traitement dans les conditions classiques à la fois du plasmide linéarisé ainsi modifié et de la séquence de nucléotides contenant l'insérat avec l'enzyme de restriction en question et la ligation, notamment en présence d'une T4 ADN-ligase des fragments entre eux pour reconstituer le plasmide contenant l'insérat dans la position ainsi choisie.

L'invention concerne encore les micro-organismes transformés comportant des protéines hybrides exposées à leur surface et contenant la séquence déterminée d'aminoacides codée par le susdit insérat. L'invention concerne enfin un procédé de fabrication de telles souches transformées, caractérisé par la transformation d'un microorganisme appartenant à la catégorie de ceux qui portent des récepteurs du phage notamment E. coli avec le vecteur tel qu'il a été défini plus haut, la sélection des souches transformées et la culture des souches transformées productrices de ladite protéine hybride sur les membranes externes de leurs cellules dans un milieu de culture approprié. De préférence, la souche mise en oeuvre est un mutant initialement résistant au phage et/ou initialement incapable de croître spontanément dans un milieu contenant une maltodextrine.

Les souches transformées sont alors sélectionnées selon qu'elles sont devenues non résistantes au phage et/ou devenues capables de croître dans un milieu contenant de la maltodextrine. Le cas échéant, la protéine hybride peut être récupérée à partir de ces micro-organismes, notamment après rupture ou lyse des parois, notamment en ayant recours à des fractionnements sur gel et, le cas échéant, en mettant en oeuvre des tests immunologiques de reconnaissance des protéines hybrides. Par exemple, on met en oeuvre des anticorps préalablement formés contre la séquence d'acides aminés ou la protéine qui la contient.

Des caractéristiques supplémentaires de l'invention apparaîtront encore au cours de la description qui suit d'un principe préféré de fabrication des vecteurs conformes à l'invention.

1°) <u>Fabrication d'un plasmide conforme à l'invention contenant un insérat formé par une séquence de nucléotides extraite du gène S du génome de l'hépatite virale B (délimitée par des extrémités de restriction BstNI).</u>

Le site nucléotidique reconnu par l'enzyme de restriction <u>Smal</u> (position 183) dans le gène <u>lam</u> B contenu dans pBBO) a été choisi pour y insérer le susdit fragment d ADN codant pour une partie de l'antigène HBs.

Ce fragment de 44 nucléotides a été obtenu par digestion du gène S du génome du virus de l'hépatite virale B par l'enzyme de restriction BstNI, aux positions nucléotidiques 111 et 155 vis-à-vis de la position 0 du site <u>EcoRI</u> dudit génome (DNA-HBV) Les extrémités de ce fragment ont ensuite été traitées par la polymérase de Klenow pour bicaténariser les extrémités monocaténaires des fragments ainsi obtenus. Aux extrémités des fragments ainsi obtenus ont été accolés, en présence d'une enzyme ligase du phage T4, deux oligo-nucléotides obtenus par synthèse chimique, dont les séquences primaires avaient été programmées pour être reconnues par <u>Bam</u> HI. Lès fragments ainsi modifiés ont été soumis à l'action de <u>BamHI</u> pour reformer des extrémités cohésives <u>BamHI</u>. Les fragments finalement obtenus ont été à nouveau traités par un ADN-ligase du phage T4. Le plasmide reconstitué et contenant l'insérat issu du gène S de DNA-HBV, placé entre une partie proximale comportant de l'ordre de 183 triplets nucléotidiques issus de <u>lam</u> B et la partie distale constituée par une séquence de <u>lam</u> B, comportant essentiellement les triplets codant pour les 238 acides aminés suivants, a été utilisé pour transformer la souche BB1, dans les conditions qui ont déjà été indiquées. La protéine hybride formée sur une membrane externe des cultures de <u>E. coli</u> transformées et induites en présence d'IPTG peut ensuite être étudiée sur le plan de ses propriétés immunologiques, pour ce qui est de sa capacité à réagir, notamment dans un test ELISA, avec un sérum anti-HBS ou dans un test tel que décrit par ITO et ses collaborateurs, pour reconnaître des fragments du gène <u>lam</u> B.

Il va de soi que l'on peut remplacer la séquence d'ADN extraite du gène S de l'hépatite virale B par toute autre séquence de nucléotides dont l'expression pourrait être recherchée dans des conditions semblables. Par exemple, on peut avoir recours à un insérat extrait du génome d'un tout autre virus par exemple à un insérat codant pour un peptide immunogène du virus de la poliomyélite ou de la fièvre aphteuse et pour un fragment d'une toxine ou toute autre protéine douée de propriétés particulières. D'une façon générale, le vecteur selon l'invention se prête à l'expression et au transport, à travers des membranes cellulaires de souches de micro-organismes ainsi transformées, de toute séquence peptidique d'origine procaryote ou eucaryote.

L'invention fournit par conséquent des moyens d'étude particulièrement intéressants des comportements réciproques des micro-organismes transformés de la séquence peptidique susceptible d'être transportée à travers la membrane externe du micro-organisme. L'invention permet en outre, grâce à l'exposition à la surface de micro-organismes de protéines de constitution déterminée, l'étude de principes vaccinants mettant en jeu par exemple une protéine virale vaccinante fixée sur la bactérie hôte (lorsque celle-ci est inoffensive) de sorte que celle-ci puisse être directement utilisée comme véhicule porteur des antigènes vaccinants que constituent ces protéines virales.

Comme il va de soi et comme il résulte d'ailleurs déjà de ce qui précède, l'invention ne se limite nullement à ceux de ses modes d'application et de réalisation qui ont été plus spécialement envisagés elle en embrasse au contraire toutes les variantes; en particulier dans ce qui précède, il a surtout été fait état du gène <u>lam</u> B ou de parties de celui-ci. Il va de soi que doivent également être considérées comme des équivalents entrant dans le champ de protection des revendications toutes autres formes d'ADN ou de fragments d'ADN capables de coder pour des polypeptides identiques ou ayant des propriétés semblables. On mentionnera à titre d'exemples, les séquences de cADN susceptibles d'être obtenues par recopie enzymatique de l'ARN messager correspondant au récepteur du phage le cas échéant à son précurseur. Il va de soi qu'un tel cADN ou les parties antérieure et postérieure correspondantes pourraient, comme il a été décrit plus haut en ce qui concerne les fragments de <u>lam</u> B, être mis sous le contrôle du promoteur en mettant en oeuvre des méthodes de recombinaison génétique bien connues des spécialistes.

L'invention n'est pas davantage limitée aux microorganismes constitués par <u>E. coli</u> On re-

marque que des techniques semblables peuvent être appliquées à des espèces différentes de micro-organismes, tels que les shighellae, lesquels sont également susceptibles de synthétiser dans les conditions qui ont été évoquées plus haut, une protéine hybride de membrane externe. D'une façon plus générale encore l'invention s'étend à toutes espèces de micro-organismes, dès lors qu'ils sont transformables par le plasmide vecteur choisi, lequel aura au préalable été inséré dans la séquence devant être exprimée et contenant l'insérat susdit entouré par les parties antérieure N-terminale et postérieure C-terminale de lam B.

Enfin, on remarquera que les techniques de clonage décrites par MANIATIS, FRITSCH et SAMBROOK dans l'ouvrage intitulé "Molecular cloning" ont été appliquées dans les exemples indiqués plus haut. On peut avoir naturellement recours à toute autre technique classique. Par exemple, on mentionnera encore les techniques décrites dans la publication déjà mentionnée de H. A. DE BOER et Coll.

## Revendications

1. Vecteur modifié par une séquence nucléotidique contenant un insérat déterminé de nucléotides codant pour une séquence déterminée d'aminoacides, cette séquence nucléotidique étant placée sous le contrôle d'un promoteur suffisamment fort pour permettre la transcription et la traduction essentiellement de la totalité de ladite séquence nucléotidique dans une souche de E. coli préalablement transformée par ledit vecteur modifié, ledit vecteur étant caractérisé en ce que la partie proximale de ladite séquence nucléotidique, vis-à-vis dudit promoteur et en amont dudit insérat déterminé de nucléotides, contient la partie antérieure du gène lam B codant pour au moins les 180 premiers aminoacides de la partie N-terminale de la protéine correspondante et, en aval dudit insérat déterminé, la partie postérieure du gène lam B, codant pour au moins les 20 derniers aminoacides de la partie C-terminale de la protéine correspondante, lesdites parties antérieures et postérieures du gène lam B ainsi que le susdit fragment de nucléotides étant respectivement en phase avec le promoteur, afin que la protéine hybride résultant de la traduction de ladite sequence sous le contrôle dudit promoteur contiene d'une part, respectivement au moins les 180 premiers et au moins les 20 derniers aminoacides de la protéine codée par le gène lam B et, d'autre part, la susdite séquence déterminée d'aminoacides.

2. Vecteur selon la revendication 1, caractérisé en ce que les susdites partie antérieure et partie postérieure du gène lam B contiennent ensemble un nombre total suffisant de nucléotides pour que de telles parties, lorsqu'elles sont placées en phase sous le contrôle d'un promoteur tac à l'intérieur d'un plasmide le contenant, soient aptes à permettre l'obtention dans une souche de E. coli préalablement transformée par ce plasmide, d'une protéine stable et non léthale pour E. coli, sur la membrane externe du micro-organisme.

3. Vecteur selon la revendication 1, caractérisé en ce que les susdites parties antérieure et postérieure du gène lam B totalisent ensemble au moins 350 triplets de nucléotides.

4. Vecteur selon la revendication 1, caractérisé en ce que les susdites parties antérieure et postérieure du gene lam B contiennent ensemble substantiellement la totalité des nucléotides du gène lam B.

5. Vecteur modifié selon l'une quelconque des revendications 1 à 4, caractérisé en ce que les premiers triplets de nucléotides, transcrits et traduits sous le contrôle du susdit promoteur, appartiennent à la partie antérieure du gène lam B.

6. Vecteur selon l'une quelconque des revendications 1 à 5, caractérisé en ce que le promoteur susdit est un promoteur tac.

7. Vecteur selon l'une quelconque des revendications 1 à 6, caractérisé en ce qu'il est constitué par un plasmide, notamment dérivé de pBR 322.

8. Micro-organisme, notamment du type E. coli, transformé par un vecteur conforme à l'une quelconque des revendications 1 à 7 et capable d'exprimer la susdite séquence déterminée de nucléotides sous la forme d'une protéine externe sur la membrane dudit micro-organisme.

9. Micro-organisme selon la revendication 8, caractérisé en ce qu'il porte sur sa membrane externe des protéines contenant la susdite séquence déterminée d'aminoacides.

10. Procédé de fabrication d'une souche de E. coli comportant des protéines hybrides exposées à l'extérieur de la membrane externe des cellules, et contenant elles-mêmes une séquence déterminée d'aminoacides, caractérisé par la transformation d'un mutant de E. coli, de préférence initialement résistant au phage et/ou initialement incapable de croître spontanément dans un milieu contenant une maltodextrine, avec un vecteur conforme à l'une quelconque des revendications 1 à 7, la sélection des souches transformées devenues non résistantes au phage λ et/ou devenues capables de croître dans un milieu contenant de la maltodextrine et la culture des souches transformées productrice de ladite protéine hybride sur leur membrane externe dans un milieu de culture approprié.

## Patentansprüche

1. Durch eine Nucleotidsequenz modifizierter Vektor enthaltend eine bestimmte Einfügung von Nucleotiden, die für eine bestimmte Aminosäuresequenz kodieren, wobei diese Nucleotidsequenz unter die Kontrolle eines Promotors gestellt ist, der genügend stark ist die Transkription und die Translation im wesentlichen der Gesamtheit der besagten Nucleotidsequenz in einem Stamm von E. coli, der vorher durch den besagten modifizierten Vektor transformiert wurde, zu erlauben, dadurch gekennzeichnet, daß der proximale Teil der besagten Nucleotidsequenz, gegenüber dem besagten Promotor und oberhalb der besagten bestimmten Einfügung von Nucleotiden den vorderen Teil des Gens lam B, weicher für wenigstens die 180 ersten Aminosäuren des N-terminalen Teils des korrespondierenden Proteins kodiert und, unterhalb der besagten bestimmten Einfügung, den hinteren Teil des Gens lam B enthält, weicher für wenigstens die 20 letzten Aminosäuren des C-terminalen Teils des korrespondierenden Proteins kodiert, wobei die besagten vorderen und hinteren Teile des Gens lam B sowie das obengenannte Nucleotidfragment jeweils in Phase mit dem Promotor sind, damit das Hybridprotein, das aus der Translation der besagten Sequenz unter der Kontrolle des besagten Promotors resultiert, einerseits wenigstens die 180 ersten und wenigstens die 20 letzten Aminosäuren des durch das Gen lam B kodierten Proteins und andererseits die obengenannte bestimmte Aminosäuresequenz enthält.

2. Vektor nach Anspruch 1, dadurch gekennzeichnet, daß der besagte vordere und der besagte hintere Teil des Gens lam B zusammen eine ausreichende Gesamtzahl an Nucleotiden enthalten, damit solche Teile, wenn sie in Phase unter die Kontrolle eines tac-Promotors im Inneren eines den Promotor enthaltenden Plasmids gestellt sind, geeignet sind in einem vorher durch dieses Plasmid transformierten Stamm von E. coli die Darstellung eines stabilen und für E. coli nicht letalen Proteins auf der Außenmembran eines Mikroorganismus zu erlauben.

3. Vektor nach Anspruch 1, dadurch gekennzeichnet, daß die besagten vorderen und hinteren Teile des Gens lam B insgesamt zusammen mindestens 350 Nucleotidtripletts zählen.

4. Vektor nach Anspruch 1, dadurch gekennzeichnet, daß die besagten vorderen und hinteren Teile des Gens lam B zusammen im wesentlichen die Gesamtheit der Nucleotide des Gens lam B enthalten.

5. Vektor nach einem der Ansprüche 1 bis 4, dadurch gekennzeichnet, daß die ersten Nucleotidtripletts transkribiert und translatiert unter der Kontrolle des genannten Promotors, zum vorderen Teil des Gens lam B gehören.

6. Vektor nach einem der Ansprüche 1 bis 5, dadurch gekennzeichnet, daß der genannte Promotor ein tac-Promotor ist.

7. Vektor nach einem der Ansprüche 1 bis 6, dadurch gekennzeichnet, daß er durch ein Plasmid gebildet ist, insbesondere von pBR 322 abgeleitet ist.

8. Mikroorganismus, insbesondere vom Typ E. coli der durch einen Vektor nach einem der Ansprüche 1 bis 7 transformiert ist und der fähig ist, die besagte bestimmte Nucleotidsequenz in Form eines externen Proteins auf der Membran des besagten Mikroorganismus zu exprimieren.

9. Microorganismus nach Anspruch 8, dadurch gekennzeichnet, daß er auf seiner Außenmembran Proteine trägt, die die besagte bestimmte Aminosäuresequenz enthalten.

10. Verfahren zur Herstellung eines Stammes von E. coli, das exponierte Hybridproteine auf der Außenmembran der Zellen enthält, und die selbst eine bestimmte Aminosäuresequenz enthalten, gekennzeichnet durch die Transformation eines Mutanten von E. coli, der vorzugsweise ursprünglich resistent gegen Phage λ und/oder ursprünglich unfähig zur Selbstvermehrung in einem ein Maltodextrin enthaltenden Milieu ist, mit einem Vektor nach einem der Ansprüche 1 bis 7, die Selektion der transformierten Stämme, die gegen Phage λ nicht-resistent werden und/oder zur Vermehrung in einem das Maltodextrin enthaltenden Milieu fähig werden, und die Kultur der transformierten Stämme zur Herstellung des besagten Hybridproteins auf ihrer Außenmembran in einem angepaßten Kulturmilieu.

## Claims

1. Vector modified by a nucleotide sequence containing a predetermined nucleotide insert coding for a predetermined amino acid, said nucleotide sequence being placed under the control of a sufficiently strong promoter to permit the transcription and translation essentially of the whole of said nucleotide sequence in a strain of E. coli previously transformed by said modified vector, said vector being characterized in that the proximal part of said nucleotide sequence, with respect to said promoter and upstream of said predetermined nucleotide insert, contains the front part of the lam B gene coding for at least the 180 first amino acids of the N-terminal part of the corresponding protein, and wherein the distal part of said nucleotide sequence, also situated downstream of said predetermined insert, comprises the rear part of the lam B gene, coding for at least the 20 last amino acids of the C-terminal part of the corresponding protein, said front and rear parts of the lam B gene as well as the abovesaid nucleotide fragment being respectively in phase with the promoter, wherein the hybrid protein resulting from the translation of said sequence under the control of said promoter contains, on the one hand, respectively at least the 180 first and at least the 20 last amino acids of the protein coded by the lam B gene and, on the other hand, the abovesaid predetermined amino acid sequence.

2. The vector of claim 1, characterized in that the abovesaid front and rear parts of the lam B gene contain together a sufficient total number of nucleotides to provide, when they are placed in phase under the control of a tac promoter inside a plasmid containing it, for the production in a E. coli strain previously transformed by said plasmid, of a protein stable and non-lethal for E.

coli, on the outer membrane thereof.

3. The vector according to claim 1, characterized in that the abovesaid front and rear parts of the lam B gene contain together at least 350 nucleotide triplets.

4. The vector of claim 1, characterized in that the abovesaid front and rear parts of the lam B gene contain together substantially all the nucleotides of the lam B gene.

5. The vector according to any one of claims 1 to 4, characterized in that the first nucleotide triplets, transcribed and translated under the control of the abovesaid promoter, belong to the front part of the lam B gene.

6. The vector according to any one of claims 1 to 5 characterized in that said promoter is a tac promoter.

7. The vector according to any one of claims 1 to 6 characterized in that it consists of a plasmid, more particularly a plasmid derived from pBR322.

8. A microorganism, more particularly of the E. coli type transformed by a vector according to any one of claims 1 to 7, and comprising on its outer membrane a hybrid protein which is an expression product of said predetermined nucleotide sequence.

9. A microorganism according to claim 8, characterized in that it bears proteins containing the abovesaid predetermined aminoacid sequence on its outer membrane.

10. Process for producing a strain of E. coli comprising hybrid proteins exposed on the outer membrane of the cells, said proteins comprising a predetermined aminoacid sequence, characterized by transforming a mutant of E. coli, preferably initially resistant to λ phage and/or initially incapable of growing spontaneously in a medium containing a maltodextrin, with a vector according to any one of claims 1 to 7, selecting the transformed strains which became non-resistant to the λ phage and/or capable of growing in a medium containing maltodextrin and culturing the transformed strains producing the said hybrid proteins on their outer membrane in a suitable culture medium.

```
                                    10                                      20
ValAspPheHisGlyTyrAlaArgSerGlyIleGlyTrpThrGlySerGlyGlyGluGlnGlnCysPheGlnThr
GTTGATTTCCACGGCTATGCACGTTCCGGTATTGGTTGGACAGGTAGCGGCGGTGAACAACAGTGTTTCCAGACT

                    30                                40                      50
ThrGlyAlaGlnSerLysTyrArgLeuGlyAsnGluCysGluThrTyrAlaGluLeuLysLeuGlyGlnGluVal
ACCGGTGCTCAAAGTAAATACCGTCTTGGCAACGAATGTGAAACTTATGCTGAATTAAAATTGGGTCAGGAAGTG

                            60                          70
TrpLysGluGlyAspLysSerPheTyrPheAspThrAsnValAlaTyrSerValAlaGlnGlnAsnAspTrpGlu
TGGAAAGAGGGCGATAAGAGCTTCTATTTCGACACTAACGTGGCCTATTCCGTCGCACAACAGAATGACTGGGAA

            80                          90                              100
AlaThrAspProAlaPheArgGluAlaAsnValGlnGlyLysAsnLeuIleGluTrpLeuProGlySerThrIle
GCTACCGATCCGGCCTTCCGTGAAGCAAACGTGCAGGGTAAAAACCTGATCGAATGGCTGCCAGGCTCCACCATC

                            110                             120
TrpAlaGlyLysArgPheTyrGlnArgHisAspValHisMetIleAspPheTyrTyrTrpAspIleSerGlyPro
TGGGCAGGTAAGCGCTTCTACCAACGTCATGACGTTCATATGATCGACTTCTACTACTGGGATATTTCTGGTCCT

            130                             140                             150
GlyAlaGlyLeuGluAsnIleAspValGlyPheGlyLysLeuSerLeuAlaAlaThrArgSerSerGluAlaGly
GGTGCCGGTCTGGAAAACATCGATGTTGGCTTCGGTAAACTCTCTCTGGCAGCAACCCGCTCCTCTGAAGCTGGT

                            160                             170
GlySerSerSerPheAlaSerAsnAsnIleTyrAspTyrThrAsnGluThrAlaAsnAspValPheAspValArg
GGTTCTTCCTCTTTCGCCAGCAACAATATTTATGACTATACCAACGAAACCGCGAACGACGTTTTCGATGTGCGT

            180                             190                             200
LeuAlaGlnMetGluIleAsnProGlyGlyThrLeuGluLeuGlyValAspTyrGlyArgAlaAsnLeuArgAsp
TTAGCGCAGATGGAAATCAACCCGGGCGGCACATTAGAACTGGGTGTCGACTACGGTCGTGCCAACTTGCGTGAT
         == ============
                                210                             220
AsnTyrArgLeuValAspGlyAlaSerLysAspGlyTrpLeuPheThrAlaGluHisThrGlnSerValLeuLys
AACTATCGTCTGGTTGATGGCGCATCGAAAGACGGCTGGTTATTCACTGCTGAACATACTCAGAGTGTCCTGAAG

            230                             240                             250
GlyPheAsnLysPheValValGlnTyrAlaThrAspSerMetThrSerGlnGlyLysGlyLeuSerGlnGlySer
GGCTTTAACAAGTTTGTTGTTCAGTACGCTACTGACTCGATGACCTCGCAGGGTAAAGGGCTGTCGCAGGGTTCT

                            260                             270
GlyValAlaPheAspAsnGluLysPheAlaTyrAsnIleAsnAsnAsnGlyHisMetLeuArgIleLeuAspHis
GGCGTTGCATTTGATAACGAAAAATTTGCCTACAATATCAACAACAACGGTCACATGCTGCGTATCCTCGACCAC

                280                             290                         300
GlyAlaIleSerMetGlyAspAsnTrpAspMetMetTyrValGlyMetTyrGlnAspIleAsnTrpAspAsnAsp
GGTGCGATCTCCATGGGCGACAACTGGGACATGATGTACGTGGGTATGTACCAGGATATCAACTGGGATAACGAC

                            310                             320
AsnGlyThrLysTrpTrpThrValGlyIleArgProMetTyrLysTrpThrProIleMetSerThrValMetGlu
AACGGCACCAAGTGGTGGACCGTCGGTATTCGCCCGATGTACAAGTGGACGCCAATCATGAGCACCGTGATGGAA

            330                             340                             350
IleGlyTyrAspAsnValGluSerGlnArgThrGlyAspLysAsnAsnGlnTyrLysIleThrLeuAlaGlnGln
ATCGGCTACGACAACGTCGAATCCCAGCGCACCGGCGACAAGAACAATCAGTACAAAATTACCCTCGCACAACAA

                            360                             370
TrpGlnAlaGlyAspSerIleTrpSerArgProAlaIleArgValPheAlaThrTyrAlaLysTrpAspGluLys
TGGCAGGCTGGCGACAGCATCTGGTCACGCCCGGCTATTCGTGTCTTCGCAACCTACGCCAAGTGGGATGAGAAA

            380                             390                             400
TrpGlyTyrAspTyrThrGlyAsnAlaAspAsnAsnAlaAsnPheGlyLysAlaValProAlaAspPheAsnGly
TGGGGGTTACGACTACACCGGTAACGCTGATAACAACGCGAACTTCGGCAAAGCCGTTCCTGCTGATTTCAACGGC

                            410                             420
GlySerPheGlyArgGlyAspSerAspGluTrpThrPheGlyAlaGlnMetGluIleTrpTrp
GGCAGCTTCGGTCGTGGCGACAGCGACGAGTGGACCTTCGGTGCCCAGATGGAAATCTGGTCG
```

1